(19) Europäisches Patentamt / European Patent Office / Office européen des brevets

(11) **EP 3 066 976 A1**

(12) **EUROPEAN PATENT APPLICATION**
published in accordance with Art. 153(4) EPC

(43) Date of publication:
**14.09.2016 Bulletin 2016/37**

(51) Int Cl.:
*A61B 5/00* *(2006.01)*    *A61B 5/107* *(2006.01)*
*G06T 1/00* *(2006.01)*

(21) Application number: **14859686.9**

(22) Date of filing: **29.09.2014**

(86) International application number:
**PCT/JP2014/075884**

(87) International publication number:
**WO 2015/068494 (14.05.2015 Gazette 2015/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority:   **05.11.2013   JP 2013229221**

(71) Applicant: **Konica Minolta, Inc.
Tokyo 100-7015 (JP)**

(72) Inventor: **MATSUDA, Shinya
Tokyo 100-7015 (JP)**

(74) Representative: **Gille Hrabal
Brucknerstrasse 20
40593 Düsseldorf (DE)**

(54)   **ORGAN IMAGE CAPTURING DEVICE**

(57)   An organ image photographing apparatus (1) includes an imaging unit (3), an image processing unit (15), and a detecting unit (17). The imaging unit (3) obtains an image by photographing a living organ. The image processing unit (15) creates a frequency distribution of image data including any of red, green, and blue color components from the photographed image of the organ obtained by the imaging unit (3), and obtains an index indicating a spread of the frequency distribution. The detecting unit (17) detects a crack in a surface of the organ based on the index.

FIG. 2

EP 3 066 976 A1

**Description**

Technical Field

The present invention relates to an organ image photographing apparatus that detects cracks in an organ surface from an image obtained by photographing a living organ.

Background Art

In Oriental medicine, there is known a diagnosis technique (tongue diagnosis) for diagnosing a health condition or a medical condition by observing tongue conditions. In tongue diagnosis, a physical condition and the degree of health are determined based on the color and shape of a tongue (to be precise, a tongue and a coating).

One of diagnosis options in tongue diagnosis is whether there are cracks (called fissures) in a tongue surface. When immune deterioration occurs due to nutritional deficiency, poor blood flow, stress, etc., the regeneration power of tongue cells decreases, causing cracks in the tongue surface. This state is called Qi deficiency or blood deficiency in Oriental medicine, and if it gets worse, anemia or malnutrition is caused.

At present, these diagnoses are made by specialized doctors. However, the diagnoses rely on the experience or intuition of the doctors and thus vary between individuals and have poor objectivity. In addition, the doctors have vague memories about the past conditions and thus cannot recognize objective changes in conditions.

In view of this, there is proposed a system that photographs a subject using a digital camera, converts features of the subject into numerical values from a photographed image, records the numerical values, and makes a diagnosis. For example, in Patent Literature 1, a tongue is photographed by a camera and an area of interest, such as the tip of the tongue, the center of the tongue, the sides of the tongue, or the root of the tongue, is extracted so that an individual health condition can be easily diagnosed based on a change in conditions in the area of interest. In addition, in Patent Literature 2, by photographing a tongue by a camera and detecting the color and gloss of the tongue separately, an index for diagnosing blood and vessel conditions is obtained. Furthermore, in Patent Literature 3, the papillae and foundation of a tongue are identified from data of a photographed image of the tongue (e.g., red (R) and green (G) image data), and if the length of pixels determined to be the foundation is greater than or equal to a certain length, then it is determined that the tongue has a crack.

Citation List

Patent Literatures

Patent Literature 1: JP 3854966 B1 (see claim 1, paragraphs [0004] and [0009], etc.)
Patent Literature 2: JP 2011-239926 A (see claim 1, paragraph [0028], etc.)
Patent Literature 3: JP 2005-137756 A (see claim 3, paragraphs [0079] to [0081], Fig. 8, etc.)

Summary of Invention

Technical Problem

However, Patent Literatures 1 and 2 do not mention the detection of cracks in the tongue surface at all.

In addition, with a crack detection method of Patent Literature 3, a crack may not be able to be detected depending on the conditions of the tongue surface (e.g., the thickness of a coating and the length of a crack). For example, when the tongue coating is thin and there is only a little difference in color between papillae and foundation, identifying papillae and foundation based on the color itself becomes difficult, and thus, a crack cannot be detected. In general, in a symptom with many cracks in the tongue surface, there are many cases with a very little coating. In such a case, cracks cannot be detected. In addition, when cracks are spread out all over the tongue and discontinuously occur, since the length of pixels determined to be the foundation is short, in such a case, too, cracks cannot be detected.

The present invention has been made to solve the above-described problems, and an object thereof is to provide an organ image photographing apparatus capable of performing detection of cracks with high accuracy, irrespective of the conditions of an organ surface.

Solution to Problem

An organ image photographing apparatus according to one aspect of the present invention is an organ image photographing apparatus including an imaging unit that obtains an image by photographing a living organ. The organ image photographing apparatus includes : an image processing unit that creates a frequency distribution of image data from the photographed image of the organ, and obtains an index indicating a spread of the frequency distribution, the photographed image being obtained by the imaging unit, and the image data including any of red, green, and blue color components; and a detecting unit that detects a crack in a surface of the organ based on the index.

Advantageous Effects of Invention

According to the above-described configuration, detection of cracks can be performed with high accuracy, irrespective of the conditions of an organ surface.

Brief Description of Drawings

Fig. 1 is a perspective view showing an external appearance of an organ image photographing apparatus according to one embodiment of the present invention.

Fig. 2 is a block diagram showing a schematic configuration of the organ image photographing apparatus.

Fig. 3 is an illustrative diagram showing a positional relationship between an illuminating unit and an imaging unit of the organ image photographing apparatus with respect to a photographing object.

Fig. 4 is an illustrative diagram showing a photographed image of a tongue obtained by the imaging unit, an edge extraction filter, and a contour of the tongue which is extracted from the photographed image using the edge extraction filter.

Fig. 5 is an illustrative diagram showing a photographed image for a case of cracks in the tongue.

Fig. 6 is an illustrative diagram showing a positional relationship between the contour of the tongue and a crack detection area.

Fig. 7 is graphs schematically showing frequency distributions of B image data which are obtained from photographed images of the tongue.

Fig. 8 is an illustrative diagram showing a relationship between the standard deviation of the frequency distribution and the observation of fissures.

Fig. 9 is a flowchart showing the flow of operation of the organ image photographing apparatus.

Fig. 10 is graphs showing spectral distributions of the tongue.

Fig. 11 is graphs schematically showing frequency distributions of R image data which are obtained from photographed images of the tongue.

Fig. 12 is graphs schematically showing frequency distributions of G image data which are obtained from photographed images of the tongue.

Description of Embodiments

One embodiment of the present invention is described as follows based on the drawings. Note that in the present specification, when a numerical value range is represented as A to B, the numerical value range includes the values of the lower limit A and the upper limit B.

[Overall configuration of an organ image photographing apparatus]

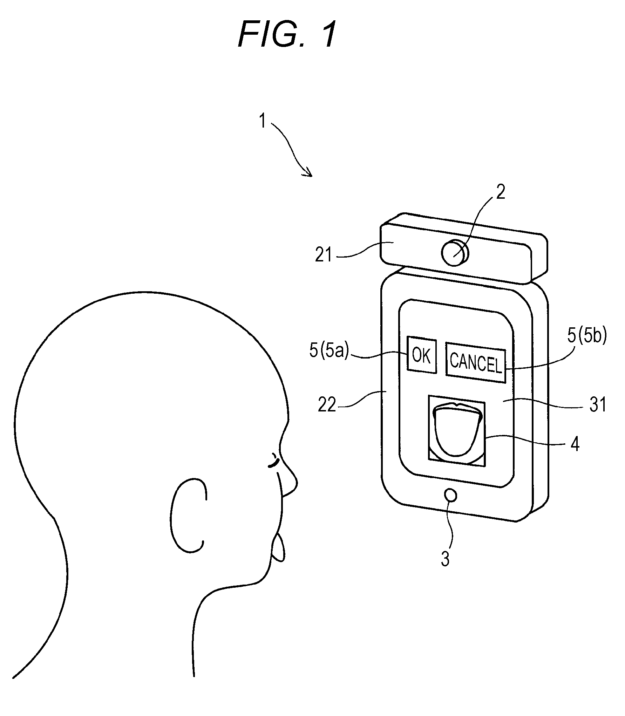

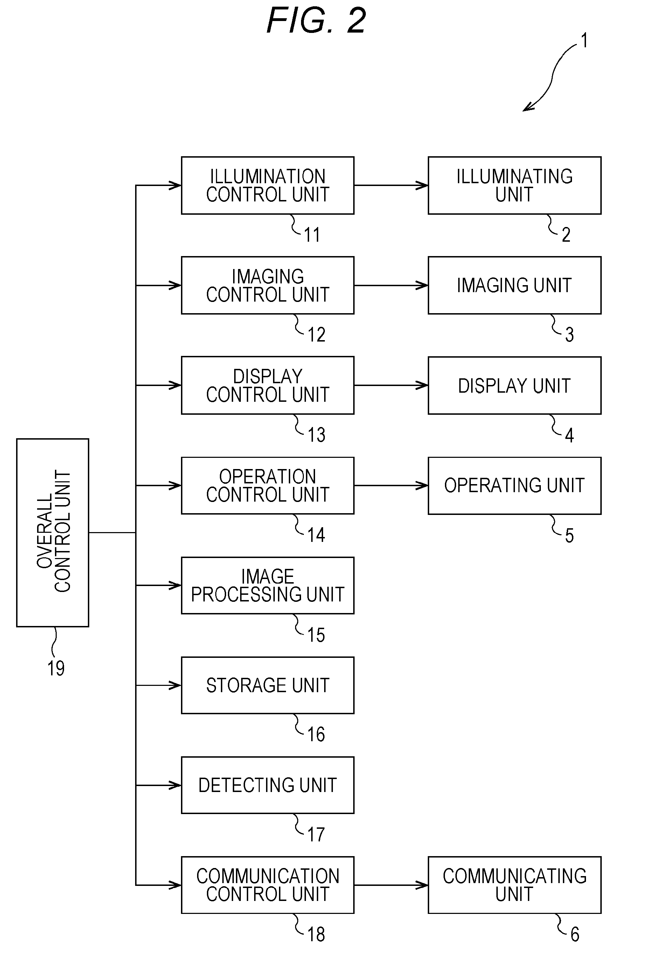

Fig. 1 is a perspective view showing an external appearance of an organ image photographing apparatus 1 of the present embodiment, and Fig. 2 is a block diagram showing a schematic configuration of the organ image photographing apparatus 1. The organ image photographing apparatus 1 is to photograph a living organ and extract information required for diagnosis of the degree of health. The following shows, as an example, the case in which a photographing object is a tongue which is a living organ.

The organ image photographing apparatus 1 includes an illuminating unit 2, an imaging unit 3, a display unit 4, an operating unit 5, and a communicating unit 6. The illuminating unit 2 is provided to a casing 21, and components other than the illuminating unit 2 (e.g., the imaging unit 3, the display unit 4, the operating unit 5, and the communicating unit 6) are provided to a casing 22. The casing 21 and the casing 22 are connected to each other so as to be relatively rotatable, but do not necessarily require rotation, and one may be completely fixed to the other. Note that the above-described illuminating unit 2 and the like may be provided to a single casing. In addition, the organ image photographing apparatus 1 may be composed of a multifunctional mobile information terminal.

The illuminating unit 2 is composed of an illuminator that illuminates the photographing object from above. For a light source of the illuminating unit 2, to improve color reproducibility, for example, a light source that emits daylight color light such as a xenon lamp is used. The brightness of the light source varies depending on the sensitivity of the imaging unit 3 or the distance to the photographing object, but as an example, brightness that allows to obtain an illuminance of the photographing object of 1000 to 10000 lx can be considered. The illuminating unit 2 includes a lighting circuit and a dimming circuit in addition to the above-described light source, and the turning on/off and dimming of the illuminating unit 2 are controlled by instructions from an illumination control unit 11.

The imaging unit 3 is to obtain an image by photographing a living organ under illumination by the illuminating unit 2, and includes an imaging lens and an area sensor (imaging device). The diaphragm of the imaging lens (the brightness of the lens), shutter speed, and focal length are set such that focus is obtained on the entire area of the photographing object. As an example, the F-number is 16, the shutter speed is 1/120 seconds, and the focal length is 20 mm.

The area sensor is composed of an imaging device, e.g., a CCD (Charge Coupled Device) or a CMOS (Complementary Metal Oxide Semiconductor), and the sensitivity, resolution, etc., of the area sensor are set such that the color and form of the photographing object can be sufficiently detected. As an example, the sensitivity is 60 db and the resolution is 10 million pixels.

Photographing by the imaging unit 3 is controlled by an imaging control unit 12. In addition, the imaging unit 3 includes a focus mechanism, a diaphragm mechanism, a drive circuit, an A/D converter circuit, and the like, which are not shown, in addition to the imaging lens and the area sensor. Control of focus and the diaphragm, control of A/D conversion, etc., are performed by instructions from the imaging control unit 12. The imaging unit 3 obtains, for example, 8-bit data ranging from 0 to 255 for each of red (R), green (G), and blue (B), as data of a photographed image.

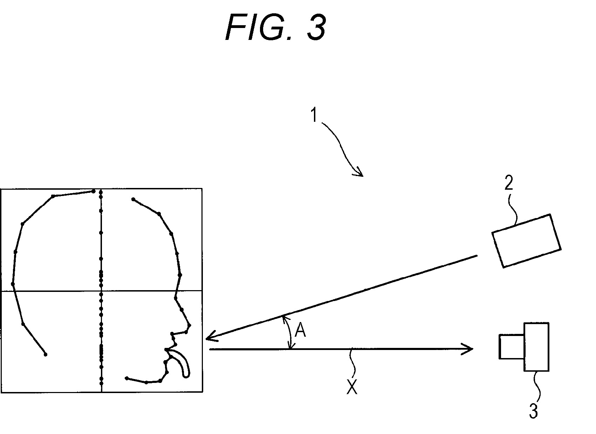

Fig. 3 is an illustrative diagram showing a positional relationship between the illuminating unit 2 and the imaging unit 3 with respect to the photographing object (tongue or face). As shown in the drawing, the imaging unit 3 is disposed so as to face right in front of the photographing object. The illuminating unit 2 is disposed so as to illuminate the photographing object at an angle A of, for example, 0° to 45° with respect to a photographing optical axis X of the imaging unit 3 which passes through the photographing object. Note that the photographing optical axis X indicates the optical axis of the imaging lens included in the imaging unit 3.

When the angle A upon illumination is large, a tongue photographable range decreases due to a shadow of the upper lip. On the other hand, when the angle A is small, washed out colors due to regular reflection increase. Taking into account the above, a preferred range of the angle A upon illumination is 15° to 30°.

The display unit 4 includes a liquid crystal panel, a backlight, a lighting circuit, and a control circuit which are not shown, and displays an image obtained by photographing performed by the imaging unit 3. In addition, the display unit 4 can also display information that is obtained from an external source through the communicating unit 6 (e.g., a diagnosis result obtained by transmitting information to an external medical institution). Display of various types of information on the display unit 4 is controlled by a display control unit 13.

The operating unit 5 is an input unit for instructing photographing performed by the imaging unit 3, and is composed of an OK button (photographing execution button) 5a and a CANCEL button 5b. In the present embodiment, the display unit 4 and the operating unit 5 are composed of a common touch panel display apparatus 31, and a display area of the display unit 4 and a display area of the operating unit 5 on the touch panel display apparatus 31 are separated from each other. Display of the operating unit 5 on the touch panel display apparatus 31 is controlled by an operation control unit 14. Note that the operating unit 5 may be composed of an input unit other than the touch panel display apparatus 31 (the operating unit 5 may be provided in a location outside the display area of the touch panel display apparatus 31).

The communicating unit 6 is an interface for transmitting data of an image obtained by the imaging unit 3 and data processed by an image processing unit 15 and a detecting unit 17 which will be described later, to an external source through a communication line (including wired and wireless), and receiving information transmitted from an external source. The transmission and reception of information performed by the communicating unit 6 are controlled by a communication control unit 18.

In addition, the organ image photographing apparatus 1 further includes the illumination control unit 11, the imaging control unit 12, the display control unit 13, the operation control unit 14, the image processing unit 15, a storage unit 16, the detecting unit 17, the communication control unit 18, and an overall control unit 19 that controls these units. As described above, the illumination control unit 11, the imaging control unit 12, the display control unit 13, the operation control unit 14, and the communication control unit 18 control the illuminating unit 2, the imaging unit 3, the display unit 4, the operating unit 5, and the communicating unit 6, respectively. The overall control unit 19 is composed of, for example, a CPU (Central Processing Unit). Note that the illumination control unit 11, the imaging control unit 12, the display control unit 13, the operation control unit 14, and the communication control unit 18 and the overall control unit 19 may be composed integrally (e.g., by a single CPU).

The image processing unit 15 creates, for any of RGB colors, a frequency distribution of image data (a distribution representing a relationship between image data of each pixel of the photographed image and the number of pixels (frequency)) from the photographed image of the organ which is obtained by the imaging unit 3, and obtains an index indicating the spread of the frequency distribution. The details of the frequency distribution will be described later.

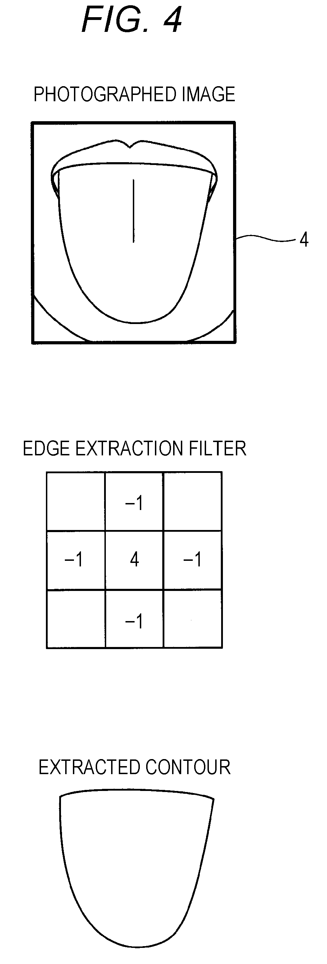

In addition, the image processing unit 15 also has a function of extracting a contour of the organ from the image obtained by the imaging unit 3. The extraction of the contour of the organ can be performed by extracting a luminance edge of the photographed image (a portion of the image where brightness suddenly changes), and the extraction of the luminance edge can be performed using, for example, an edge extraction filter such as that shown in Fig. 4. The edge extraction filter is a filter that assigns weights to pixels near a focused pixel when primary differentiation is performed (when a difference of image data is taken between adjacent pixels).

By using such an edge extraction filter, a difference of image data is taken between a focused pixel and pixels near the focused pixel for, for example, G image data of each pixel of the photographed image, and pixels whose difference values exceed a predetermined threshold value are extracted, by which pixels that serve as a luminance edge can be extracted. An area around the tongue has a luminance difference caused by a shadow thereof, and thus, by extracting pixels serving as a luminance edge in the above-described manner, a contour of the tongue can be extracted. Note that although here G image data having the largest influence on luminance is used for computation, R or B image data may be used.

Note that in Oriental medicine a white spotted portion seen at the central portion of the tongue is called a coating, and the color thereof is called a coating color. Note also that the color of a red portion of the tongue other than the above is called a tongue color.

The storage unit 16 is a memory that stores data of an image obtained by the imaging unit 3, data obtained by the image processing unit 15 and the detecting unit 17, information received from an external source, etc., and stores a program for allowing the above-described various types of control units to operate.

The detecting unit 17 detects cracks in the organ surface based on the index indicating the spread of the frequency distribution which is obtained by the image processing unit 15. Particularly, the detecting unit 17 converts the extent of cracks into a numerical value based on the index, and determines fissures in tongue diagnosis based on the numerical value. Note that the details of the detection of cracks and the determination of fissures will be described later.

[Setting of a crack detection area]

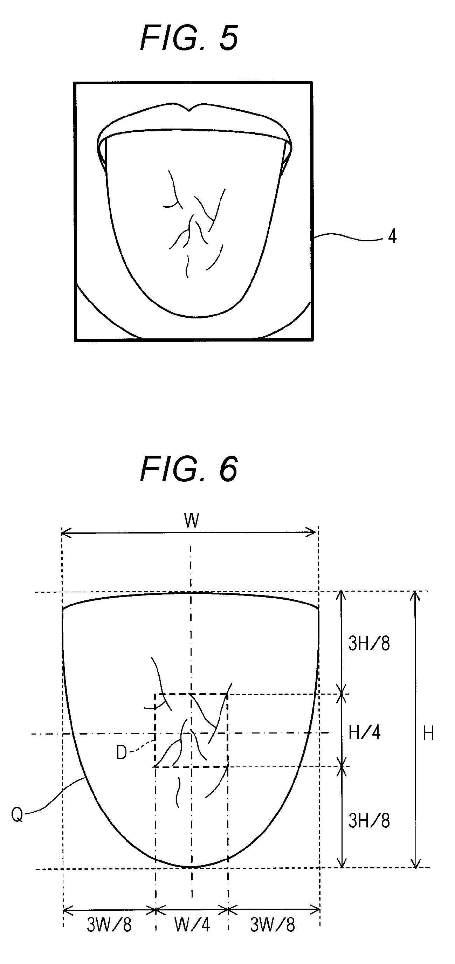

Fig. 5 shows a photographed image of a tongue with cracks in a surface thereof. When the tongue is photographed, the tongue is stuck out of the mouth forward. A surface of the stuck-out tongue on the upper lip side is photographed by the imaging unit 3. In general, cracks in the tongue surface often occur around the center of the tongue. Thus, in the present embodiment, a central portion of the tongue in up, down, left, and right directions (an area including the center in the up, down, left, and right directions) in the photographed image is set as a detection area suitable for detection of cracks.

More specifically, as shown in Fig. 6, the image processing unit 15 detects the upper and lower edges and left and right edges of the tongue from a contour Q of the tongue which is extracted from the photographed image by the above-described technique, detects a length of the tongue in the up-down direction (longitudinal dimension) H and a width in the left-right direction (transverse dimension) W, and sets, as a crack detection area D, a central area of the tongue with H/4 in the longitudinal direction and W/4 in the transverse direction which is determined by a dimensional relationship shown in Fig. 6.

Note that the crack detection area D may be set without extracting the contour Q of the tongue in the above-described manner. For example, by displaying on the display unit 4 a frame line for guiding the tongue to an appropriate photographing position so that photographing can be performed with the tongue guided to an appropriate position for each living body, a predetermined area (central area) in the photographed image of the tongue can be immediately set as the crack detection area D. In this case, since the process of extracting a contour Q becomes unnecessary, a reduction in the time required to detect cracks, simplification of a circuit configuration of the image processing unit 15, etc., can be further achieved.

[Method of detecting cracks]

When there are cracks in the surface of the tongue, the foundation of the tongue appears more compared to the case of no cracks. Thus, a range of values that can be taken by image data of pixels composing the foundation increases for all of RGB. Hence, when a frequency distribution of image data of a photographed image is created, the width of the frequency distribution increases. Particularly, since the foundation intensely represents the color of blood, for R and B which are included in the color of blood in large proportion, the width of a frequency distribution increases remarkably compared to the case of no cracks. It is known that such a tendency appears irrespective of the thickness of a coating on the tongue surface or the lengths of cracks.

Hence, in the present embodiment, the image processing unit 15 creates, for example, a frequency distribution of B image data from a photographed image of a tongue (particularly, an image of the above-described detection area D) and obtains, by computation, a standard deviation $\sigma$ indicating variation in the image data, as an index indicating the spread of the frequency distribution. The standard deviation $\sigma$ is the positive square root of variance $\sigma^2$ which is represented by the following equation when the values of the image data take N values $x_1$, $x_2$, ... $x_N$.

[Equation 1]

$$\sigma^2 = \frac{1}{N} \sum_{i=1}^{N} (x_i - m)^2$$

where

$$m = \frac{1}{N} \sum_{i=1}^{N} x_i$$

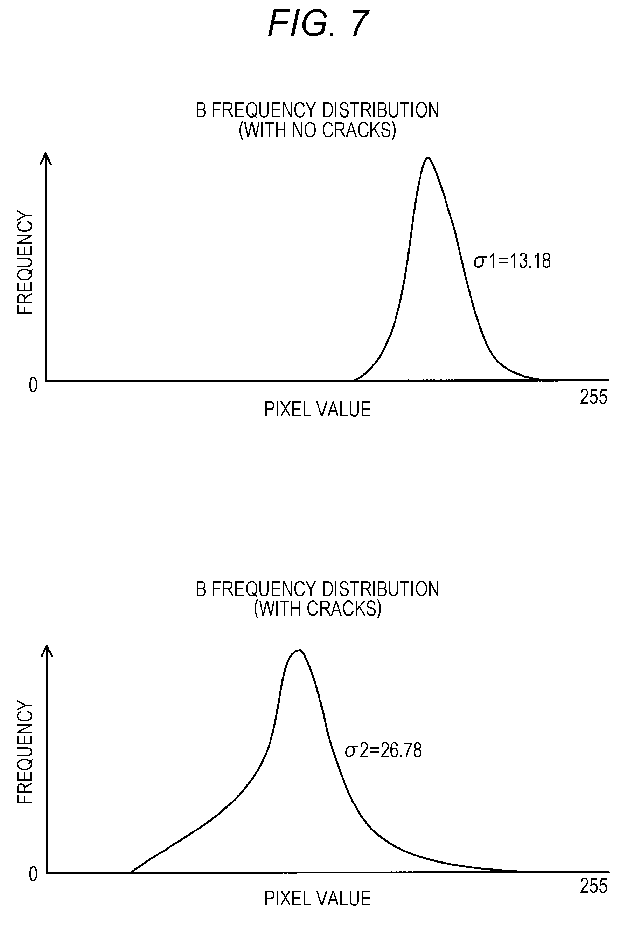

Fig. 7 schematically shows frequency distributions of B image data which are created by the image processing unit 15, and the frequency distribution at the top shows the case of no cracks in the tongue surface, and the frequency distribution at the bottom shows the case of cracks in the tongue surface. Note that the horizontal axes of the frequency distributions represent B pixel value (image data) and the vertical axes represent frequency (the number of pixels). The pixel values range from 0 to 255, and the larger the pixel value the brighter the color.

When the standard deviation $\sigma 1$ of the frequency distribution at the top is determined, $\sigma 1=13.18$ is obtained. On the other hand, when the standard deviation $\sigma 2$ of the frequency distribution at the bottom is determined, $\sigma 2=26.78$ is obtained. From this fact, it can be seen that when there are cracks in the tongue surface, the standard deviation $\sigma$ increases compared to the case of no cracks, and the width of the frequency distribution increases. For information, when a mean value $m1$ of the pixel values of the frequency distribution at the top is determined, $m1=177.71$ is obtained, and when a mean value m2 of the pixel values of the frequency distribution at the bottom is determined, m2=112.75 is obtained.

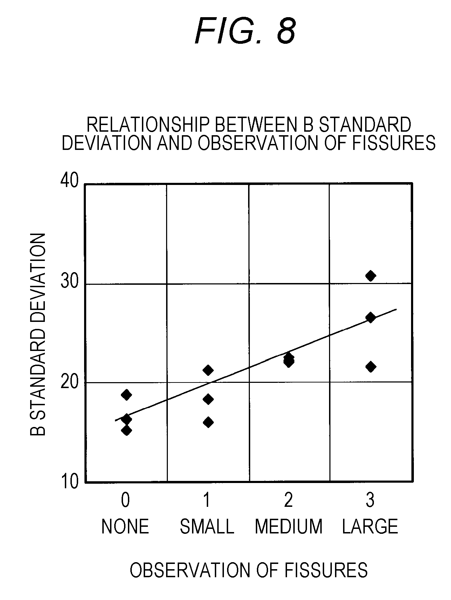

In addition, Fig. 8 shows a relationship between the standard deviations of respective frequency distributions obtained when the tongues of a plurality of people are photographed as samples and the frequency distributions of B image data included in respective images of detection areas D of photographed images are created, and observations of fissures obtained when an herbalist has actually made a tongue diagnosis of the respective tongues. From the drawing, it can be seen that there is a high correlation between the standard deviations of the frequency distributions and the herbalist's observations. Specifically, it can be said that the larger the standard deviation the larger the number of fissures, and the smaller the standard deviation the smaller the number of fissures. Note that a correlation coefficient indicating the degree of correlation between the standard deviations of the frequency distributions and the herbalist's observations has a value as high as 0.84.

Therefore, the detecting unit 17 converts the extent of cracks into a numerical value based on the standard deviation of the frequency distribution which is obtained by the image processing unit 15, and determines fissures in tongue diagnosis based on the numerical value. By this, the detecting unit 17 can make such a determination with high accuracy. For example, as shown in Fig. 8, in the case in which the extent of cracks is converted into numerical values at four levels "0" to "3", when the standard deviation of the frequency distribution is as small as 18 or less, the extent of cracks is "0" and it is determined to be "no fissures" based on the numerical value. Note that the correlation of Fig. 8 may be stored as a table in the storage unit 16, and the detecting unit 17 may determine the extent of cracks (fissures) in the tongue surface from a standard deviation of a frequency distribution by referring to the table.

[Control flow]

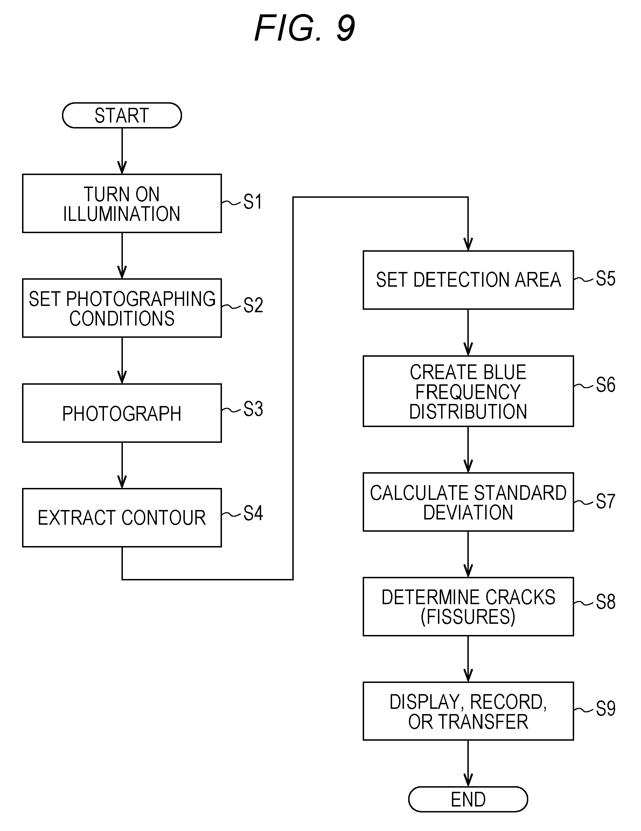

Fig. 9 is a flowchart showing the flow of operation of the organ image photographing apparatus 1 of the present embodiment. When the organ image photographing apparatus 1 accepts a photographing instruction by the operating unit 5 or an input unit which is not shown, the illumination control unit 11 turns on the illuminating unit 2 (S1), and performs the setting of photographing conditions such as illuminance (S2). When the setting of photographing conditions is completed, the imaging control unit 12 controls the imaging unit 3 to photograph a tongue which is a photographing object (S3).

When the photographing is completed, the image processing unit 15 extract a contour Q of the tongue from a photographed image of the tongue (S4). Then, the image processing unit 15 detects the upper and lower edges and left and right edges of the tongue from the extracted contour Q, and sets a crack detection area D with reference to the contour Q (S5). Then, the image processing unit 15 extracts B image data from each pixel in the set detection area D to create a frequency distribution (S6), and determines a standard deviation σ (S7).

Subsequently, the detecting unit 17 represents the extent of cracks by any of the numerical values "0" to "3" based on the obtained standard deviation σ and thereby detects whether there are cracks, and determines the extent of fissures based on the numerical value and thereby diagnoses the degree of health of a subject (S8). At this time, the detecting unit 17 may, as described above, determine the extent of cracks (fissures) corresponding to the standard deviation σ from the table stored in the storage unit 16. A result of the determination of fissures and a result of the diagnosis of the degree of health of a user are displayed on the display unit 4 and, if necessary, the results are outputted (recorded) to an output apparatus which is not shown or transferred to an external source through the communicating unit 6 (S9). Note that a result of the detection of cracks may be converted into a numerical value and the numerical value may be transmitted to an external source, and the external source may determine the extent of fissures and thereby diagnose the degree of health of the subject.

As described above, in the present embodiment, the image processing unit 15 creates a frequency distribution of B image data from a photographed image of a tongue and determines a standard deviation σ which is an index indicating the spread of the frequency distribution, and the detecting unit 17 detects cracks in a tongue surface based on the standard deviation σ. When there are cracks in the tongue surface, the width of the frequency distribution increases remarkably compared to the case of no cracks, and this tendency appears irrespective of the thickness of a coating on the tongue surface or the lengths of cracks. Accordingly, detection of cracks can be performed with high accuracy, irrespective of the thickness of a coating or the lengths of cracks.

In addition, when the organ is a tongue, the detecting unit 17 converts the extent of cracks into a numerical value based on the standard deviation σ of the frequency distribution, and thus, can easily determine fissures in tongue diagnosis based on the numerical value of the cracks.

In addition, the image processing unit 15 sets a central portion of the tongue in the photographed image as a crack detection area D, and creates a frequency distribution of image data for the set detection area D. By this, the detecting unit 17 can detect cracks occurring in the central portion of the tongue with high accuracy based on the standard deviation σ of the frequency distribution. Particularly, when the organ is a tongue, since cracks often occur in the central portion of the tongue, cracks are securely detected, enabling to improve the accuracy of diagnosis of the degree of health based on the detected cracks. In addition, since the image processing unit 15 creates a frequency distribution of image data for a partial area (detection area D) instead of for the whole tongue, the amount of data to be handled is reduced significantly, enabling to reduce the time required to create a frequency distribution and calculate a standard deviation $\sigma$.

In addition, since the standard deviation $\sigma$ is a numerical value indicating variation in image data, by using the standard deviation $\sigma$ as an index indicating the spread of the frequency distribution, the spread of the frequency distribution can be securely grasped. Note that for the index, in addition to the standard deviation $\sigma$, for example, the full width at half maximum of the frequency distribution or the width of image data ranging from a minimum value to a maximum value can also be used. That is, even if the full width at half maximum or the like is used as the index, the extent of the spread of the frequency distribution is grasped based on the index, enabling to detect whether there are cracks.

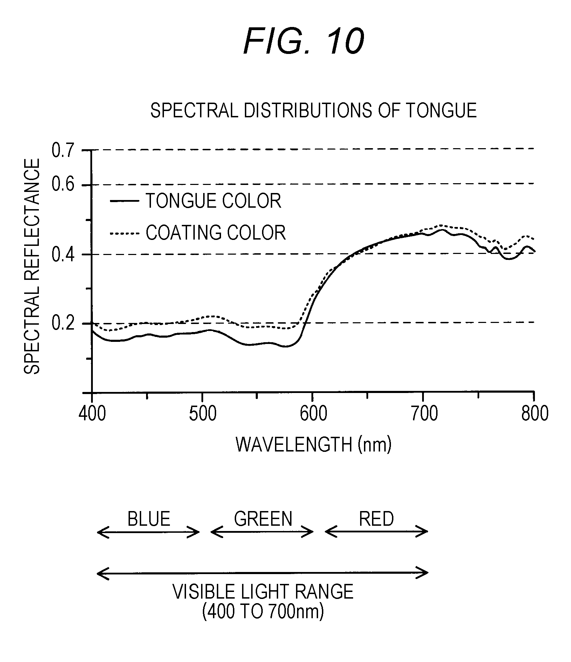

Meanwhile, Fig. 10 is graphs showing spectral distributions of a tongue. Since the tongue is mucosal integrity and thus does not have a surface skin, for the color of the tongue, the color of blood appears. The blood contains R components (a wavelength of 600 nm to 700 nm) in large proportion and B components (a wavelength of 500 nm or less) in small proportion. In addition, when the color of the tongue is light, the proportion of R components decreases, and when the color of the tongue is dark, the proportion of R components increases.

On the other hand, the coating is formed of cornified papillary cells and ranges from white to yellow in color. When the coating is thin, the color of the tongue serving as the foundation appears and thus, as shown in the drawing, the proportion of R components is high. When the coating is white and dark, the proportion of G components (a wavelength of 500 nm to 600 nm) increases.

Although the colors of the tongue and coating change in the above-described manner depending on the physical condition of the living body or individual differences, there is only a little change in B components.

Therefore, as in the present embodiment, by using a frequency distribution of B image data as a frequency distribution of a photographed image of a tongue, cracks in a tongue surface can be detected with high accuracy while the influence of the physical condition of the living body or individual differences is suppressed.

[Other methods of detecting cracks]

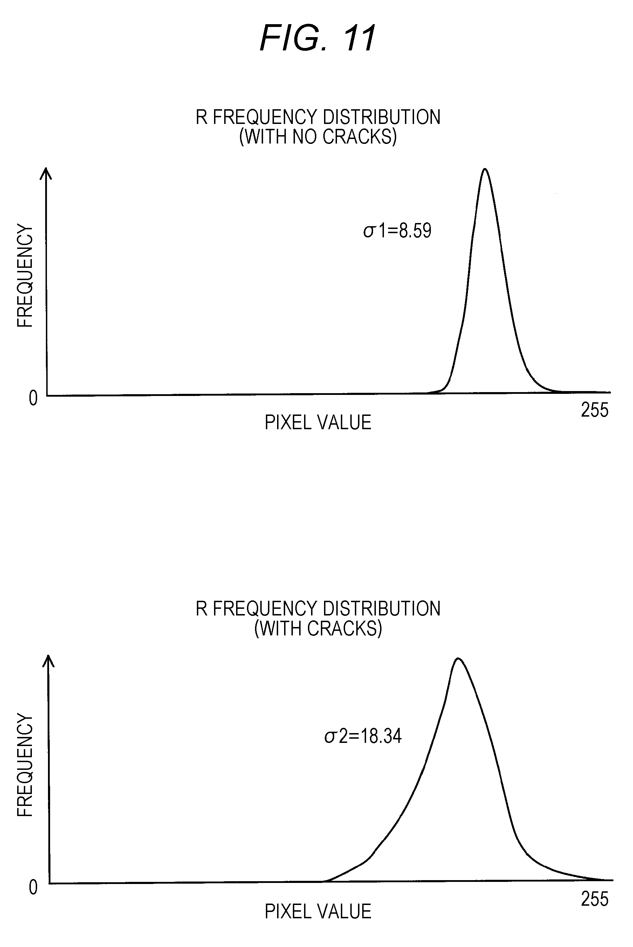

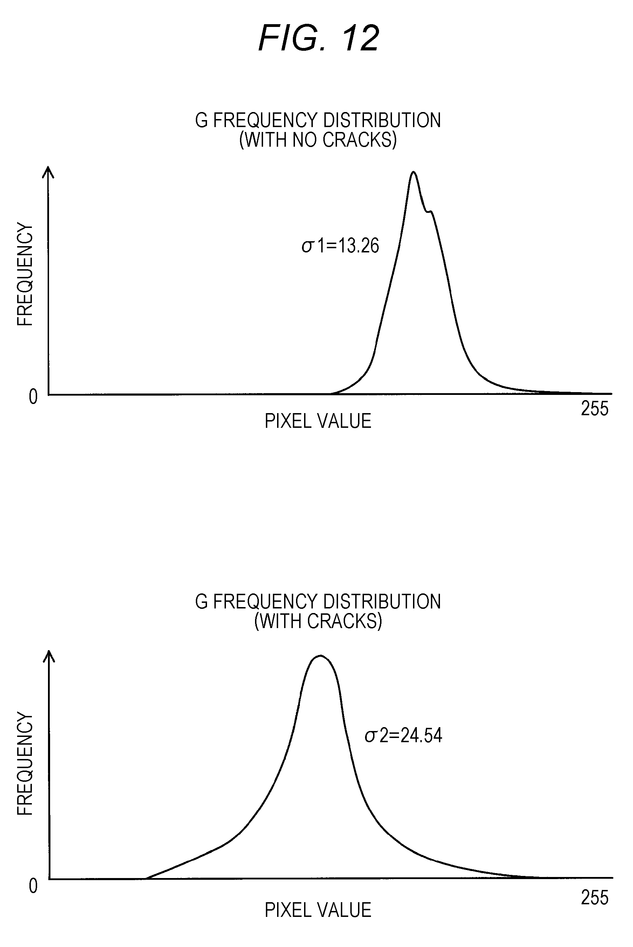

Figs. 11 and 12 schematically show frequency distributions of R and G image data, respectively, which are created by the image processing unit 15 from photographed images of tongues, and the frequency distributions at the top show the case of no cracks in the tongue surface, and the frequency distributions at the bottom show the case of cracks in the tongue surface.

When the standard deviations $\sigma1$ and $\sigma2$ of the frequency distributions at the top and bottom in Fig. 11 are determined, $\sigma1=8.59$ and $\sigma2=18.34$ are obtained. For information, when the mean values m1 and m2 of the pixel values of the frequency distributions at the top and bottom are determined, m1=200.98 and m2=184.95 are obtained.

Likewise, when the standard deviations $\sigma1$ and $\sigma2$ of the frequency distributions at the top and bottom in Fig. 12 are determined, $\sigma1=13.26$ and $\sigma2=24.54$ are obtained. For information, when the mean values m1 and m2 of the pixel values of the frequency distributions at the top and bottom are determined, m1=168.46 and m2=121.08 are obtained.

From the above, it can be seen that in the R and G frequency distributions, too, as in the case of the B frequency distributions, when there are cracks in the tongue surface, the standard deviation increases compared to the case of no cracks, and the width of the frequency distribution increases.

Therefore, even if the image processing unit 15 creates a frequency distribution of R or G image data from a photographed image of a tongue and obtains a standard deviation $\sigma$, and the detecting unit 17 detects cracks based on the standard deviation $\sigma$, detection of cracks can be performed with high accuracy, irrespective of the thickness of a coating or the lengths of cracks.

Note that G image data may possibly include not only information on cracks in the tongue surface but also information on gloss, and thus, in a frequency distribution of the G image data the information on gloss may be possibly superimposed, as noise, on the information on cracks. Hence, when cracks are detected based on the G image data, in order to reduce the influence of regular reflection of illuminated light from the illuminating unit 2 (the influence of gloss), it is desirable to set, for example, an area located slightly below the central portion of the tongue as a crack detection area D, and create a frequency distribution of image data for the detection area D to detect cracks.

In addition, the frequency distribution of image data to be used may be a frequency distribution of image data including any of RGB color components, instead of a frequency distribution of image data of any of RGB colors. For example, the image processing unit 15 may create a frequency distribution of pixel values where R and G color components are mixed, and obtain an index indicating the spread of the frequency distribution, and the detecting unit 17 may detect cracks based on the index. In this case, for the pixel value on the horizontal axes of the frequency distributions corresponding to that in Fig. 7, a total value or mean value of R and G pixel values may be used. In addition, a frequency distribution may be created using, as pixel values, other color combinations of RGB, to detect cracks.

[Others]

Although the above describes the case in which a photographing object is a human tongue, as long as the photographing object is a living body (living thing), the photographing object does not need to be a human and may be an animal other than the human. For example, even if the tongue is of an animal such as a pet or a domestic animal, by applying the technique of the present embodiment, cracks in a tongue surface can be detected and a diagnosis can be made based on a result of the detection. In this case, bad physical condition of animals that cannot perform communication can be determined promptly and accurately.

In addition, a living organ serving as a photographing object is not limited to a tongue. For example, the living organ may be oral parts such as lips and gums, stomach or intestinal lining, or the backside of eyelids. In addition, it is also possible to detect cracks in an organ surface simultaneously with an examination at each diagnosis and treatment department.

The organ image photographing apparatus described above can be represented as follows, by which the following functions and effects are provided.

The organ image photographing apparatus described above is an organ image photographing apparatus including an imaging unit that obtains an image by photographing a living organ. The organ image photographing apparatus includes: an image processing unit that creates a frequency distribution of image data from the photographed image of the organ, and obtains an index indicating a spread of the frequency distribution, the photographed image being obtained by the imaging unit, and the image data including any of red, green, and blue color components; and a detecting unit that detects a crack in a surface of the organ based on the index.

If there are cracks in the organ surface, for all of the red (R), green (G), and blue (B) colors included in the photographed image of the organ, when a frequency distribution of image data is created, the spread of the frequency distribution becomes relatively large compared to the case of no cracks. This is because, when there are cracks in the organ surface, a foundation (groove) portion of the organ appears more, and thus, the width of image data of colors (RGB) included in the foundation (the range of values that can be taken) increases. Such a tendency appears irrespective of the conditions of the organ surface (e.g., the thickness of a coating or the lengths of cracks).

Therefore, by the image processing unit creating a frequency distribution of image data including any of RGB color components from the photographed image of the organ and obtaining an index (e.g., a standard deviation) indicating the spread of the frequency distribution, and by the detecting unit detecting cracks in the organ surface based on the index, detection of cracks can be performed with high accuracy, irrespective of the conditions of the organ surface.

The organ may be a tongue, and the detecting unit may convert an extent of the crack into a numerical value based on the index, and determine a fissure in tongue diagnosis based on the numerical value. In this case, a determination of fissures in tongue diagnosis can be easily made based on the numerical value of cracks.

It is desirable that the frequency distribution be a frequency distribution of blue image data. Although the conditions of the organ surface (e.g., the color of a tongue or a coating) change depending on the physical condition of the living body or individual differences, there is only a little change in B image data. Hence, by using a frequency distribution of B image data of the photographed image of the organ, cracks in the organ surface can be detected with high accuracy while the influence of the physical condition of the living body or individual differences is suppressed.

It is desirable that the image processing unit set a central portion of the organ in the photographed image as a crack detection area, and create a frequency distribution of image data for the set detection area. In this case, cracks occurring in the central portion of the organ can be detected with high accuracy. In addition, since a frequency distribution of image data for a partial area instead of for the whole organ is created, the amount of data to be handled is reduced, enabling to reduce the time required to create a frequency distribution and calculate a standard deviation.

The index may be a standard deviation of the frequency distribution. The standard deviation is a numerical value indicating variation in image data and thus is suitable as an index indicating the spread of the frequency distribution.

The organ may be a tongue, the index may be a standard deviation of the frequency distribution, and the detecting unit may determine an extent of the crack by referring to a table indicating a relationship between the standard deviation and an observation of the crack obtained when an herbalist has actually made a tongue diagnosis. In this case, a determination of cracks that matches an herbalist's observation can be made from the obtained standard deviation, and thus, the reliability of the determination can be improved.

Industrial Applicability

The present invention can be used for an apparatus that detects cracks in an organ surface from an image obtained by photographing a living organ.

Reference Signs List

1      ORGAN IMAGE PHOTOGRAPHING APPARATUS
3      IMAGING UNIT
15     IMAGE PROCESSING UNIT
17     DETECTING UNIT
D     DETECTION AREA

**Claims**

1. An organ image photographing apparatus including an imaging unit that obtains an image by photographing a living organ, the apparatus comprising:

an image processing unit that creates a frequency distribution of image data from the photographed image of the organ, and obtains an index indicating a spread of the frequency distribution, the photographed image being obtained by the imaging unit, and the image data including any of red, green, and blue color components; and
a detecting unit that detects a crack in a surface of the organ based on the index.

2. The organ image photographing apparatus according to claim 1, wherein
the organ is a tongue, and
the detecting unit converts an extent of the crack into a numerical value based on the index, and determines a fissure in tongue diagnosis based on the numerical value.

3. The organ image photographing apparatus according to claim 1 or 2, wherein the frequency distribution is a frequency distribution of blue image data.

4. The organ image photographing apparatus according to any one of claims 1 to 3, wherein the image processing unit sets a central portion of the organ in the photographed image as a crack detection area, and creates a frequency distribution of image data for the set detection area.

5. The organ image photographing apparatus according to any one of claims 1 to 4, wherein the index is a standard deviation of the frequency distribution.

6. The organ image photographing apparatus according to claim 1, wherein
the organ is a tongue,
the index is a standard deviation of the frequency distribution, and
the detecting unit determines an extent of the crack by referring to a table indicating a relationship between the standard deviation and an observation of the crack obtained when an herbalist has actually made a tongue diagnosis.

## FIG. 1

# FIG. 2

1

```
                    ┌─────────────────┐        ┌─────────────────┐
                    │  ILLUMINATION   │───────▶│   ILLUMINATING  │
                    │  CONTROL UNIT   │        │      UNIT       │
                    └─────────────────┘        └─────────────────┘
                             11                         2

                    ┌─────────────────┐        ┌─────────────────┐
                    │    IMAGING      │───────▶│   IMAGING UNIT  │
                    │  CONTROL UNIT   │        │                 │
                    └─────────────────┘        └─────────────────┘
                             12                         3

                    ┌─────────────────┐        ┌─────────────────┐
                    │     DISPLAY     │───────▶│   DISPLAY UNIT  │
                    │  CONTROL UNIT   │        │                 │
                    └─────────────────┘        └─────────────────┘
                             13                         4

                    ┌─────────────────┐        ┌─────────────────┐
┌──────────┐        │    OPERATION    │───────▶│  OPERATING UNIT │
│ OVERALL  │        │  CONTROL UNIT   │        │                 │
│ CONTROL  │        └─────────────────┘        └─────────────────┘
│  UNIT    │                 14                         5
└──────────┘
      19            ┌─────────────────┐
                    │     IMAGE       │
                    │ PROCESSING UNIT │
                    └─────────────────┘
                             15

                    ┌─────────────────┐
                    │  STORAGE UNIT   │
                    └─────────────────┘
                             16

                    ┌─────────────────┐
                    │ DETECTING UNIT  │
                    └─────────────────┘
                             17

                    ┌─────────────────┐        ┌─────────────────┐
                    │  COMMUNICATION  │───────▶│  COMMUNICATING  │
                    │  CONTROL UNIT   │        │      UNIT       │
                    └─────────────────┘        └─────────────────┘
                             18                         6
```

# FIG. 3

# FIG. 4

PHOTOGRAPHED IMAGE

4

EDGE EXTRACTION FILTER

| | | |
|---|---|---|
| | −1 | |
| −1 | 4 | −1 |
| | −1 | |

EXTRACTED CONTOUR

# FIG. 5

4

# FIG. 6

W

3H/8

H/4

H

3H/8

Q

D

3W/8    W/4    3W/8

# FIG. 7

B FREQUENCY DISTRIBUTION
(WITH NO CRACKS)

$\sigma 1 = 13.18$

B FREQUENCY DISTRIBUTION
(WITH CRACKS)

$\sigma 2 = 26.78$

# FIG. 8

RELATIONSHIP BETWEEN B STANDARD
DEVIATION AND OBSERVATION OF FISSURES

# FIG. 9

```
              START
                │
                ▼
        ┌──────────────┐
        │   TURN ON     │  ～S1
        │ ILLUMINATION  │
        └──────────────┘
                │
                ▼
        ┌──────────────┐
        │SET PHOTOGRAPHING│  ～S2
        │  CONDITIONS   │
        └──────────────┘
                │
                ▼
        ┌──────────────┐
        │  PHOTOGRAPH   │  ～S3
        └──────────────┘
                │
                ▼
        ┌──────────────┐
        │EXTRACT CONTOUR│  ～S4
        └──────────────┘
```

```
        ┌──────────────┐
        │SET DETECTION AREA│  ～S5
        └──────────────┘
                │
                ▼
        ┌──────────────┐
        │  CREATE BLUE  │  ～S6
        │  FREQUENCY    │
        │ DISTRIBUTION  │
        └──────────────┘
                │
                ▼
        ┌──────────────┐
        │CALCULATE STANDARD│  ～S7
        │  DEVIATION    │
        └──────────────┘
                │
                ▼
        ┌──────────────┐
        │DETERMINE CRACKS│  ～S8
        │  (FISSURES)   │
        └──────────────┘
                │
                ▼
        ┌──────────────┐
        │DISPLAY, RECORD,│  ～S9
        │  OR TRANSFER  │
        └──────────────┘
                │
                ▼
              END
```

# FIG. 10

## SPECTRAL DISTRIBUTIONS OF TONGUE

# FIG. 11

R FREQUENCY DISTRIBUTION
(WITH NO CRACKS)

$\sigma 1 = 8.59$

FREQUENCY

0

PIXEL VALUE

255

R FREQUENCY DISTRIBUTION
(WITH CRACKS)

$\sigma 2 = 18.34$

FREQUENCY

0

PIXEL VALUE

255

# FIG. 12

G FREQUENCY DISTRIBUTION
(WITH NO CRACKS)

$\sigma 1 = 13.26$

FREQUENCY

0

PIXEL VALUE

255

G FREQUENCY DISTRIBUTION
(WITH CRACKS)

$\sigma 2 = 24.54$

FREQUENCY

0

PIXEL VALUE

255

**EP 3 066 976 A1**

## INTERNATIONAL SEARCH REPORT

International application No.

PCT/JP2014/075884

### A. CLASSIFICATION OF SUBJECT MATTER
*A61B5/00*(2006.01)i, *A61B5/107*(2006.01)i, *G06T1/00*(2006.01)i

According to International Patent Classification (IPC) or to both national classification and IPC

### B. FIELDS SEARCHED

Minimum documentation searched (classification system followed by classification symbols)
A61B5/00, A61B5/107, G06T1/00

Documentation searched other than minimum documentation to the extent that such documents are included in the fields searched
Jitsuyo Shinan Koho          1922–1996    Jitsuyo Shinan Toroku Koho    1996–2014
Kokai Jitsuyo Shinan Koho    1971–2014    Toroku Jitsuyo Shinan Koho    1994–2014

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2005-137756 A  (Konica Minolta Holdings, Inc.), 02 June 2005 (02.06.2005), entire text; all drawings (Family: none) | 1-6 |
| A | CN 102194121 A  (Tellyes Scientific Co., Ltd.), 21 September 2011 (21.09.2011), entire text; all drawings (Family: none) | 1-6 |
| A | JP 2009-082338 A  (Masahiro NAKAGAWA), 23 April 2009 (23.04.2009), entire text; all drawings (Family: none) | 1-6 |

☐ Further documents are listed in the continuation of Box C.    ☐ See patent family annex.

| | |
|---|---|
| *    Special categories of cited documents:<br>"A"  document defining the general state of the art which is not considered    to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search<br>    17 November, 2014 (17.11.14) | Date of mailing of the international search report<br>    02 December, 2014 (02.12.14) |
|---|---|
| Name and mailing address of the ISA/<br>    Japanese Patent Office | Authorized officer |
| Facsimile No. | Telephone No. |

Form PCT/ISA/210 (second sheet) (July 2009)

**EP 3 066 976 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3854966 B
- JP 2011239926 A

- JP 2005137756 A